# FASCICULE DE BREVET EUROPEEN

(11) **EP 0 739 875 B1**
(45) Date de publication et mention de la délivrance du brevet: **04.08.1999**
(21) Numéro de dépôt: 96200952.8
(22) Date de dépôt: 15.04.1996
(51) Int. Cl.: C07C 17/21, C07C 19/08

(54) **Procédé pour la préparation de 1,1-difluoroéthane**
Verfahren zur Herstellung von 1,1-Difluorethan
Process for the preparation of 1,1-difluoroethane

(30) Priorité: 24.04.1995 FR 9504962
(43) Date de publication de la demande: 30.10.1996
(73) Titulaire: SOLVAY (Société Anonyme), 1050 Bruxelles (BE)
(72) Inventeur: Grunchard, Frans, 1060 Bruxelles (BE); Janssens, Francine, 1800 Vilvoorde (BE); Wilmet, Vincent, 1301 Wavre (BE)
(74) Mandataire: Jacques, Philippe

(56) Documents cités:
- EP-A- 0 637 579

## Description

La présente invention concerne un procédé pour la préparation de 1,1-difluoroéthane au départ de chlorure de vinyle, réalisé en phase liquide.

La demande de brevet EP-A-0637579 divulgue un procédé d'obtention de 1,1-difluoroéthane par réaction entre du chlorure de vinyle et du fluorure d'hydrogène en phase liquide en présence d'un catalyseur d'hydrofluoration et d'un hydrocarbure halogéné saturé, le 1,2-dichloroéthane, le 1,2,3-trichloropropane, le 1-chloro-1-fluoroéthane, le 1,1-difluoroéthane, le 1,1-dichloroéthane et le 1,3-dichloro-1-fluorobutane et leurs mélanges étant préférés.

Dans ce procédé connu, une partie du chlorure de vinyle est convertie en sous-produits halogénés lourds, dont l'accumulation provoque une désactivation progressive du catalyseur au cours du temps. En outre, les produits de réaction quittant le réacteur sont inévitablement contaminés par du chlorure de vinyle, en quantité telle que son élimination peut s'avérer difficile.

La présente invention a pour but de perfectionner le procédé connu précité, de manière à remédier à la désactivation du catalyseur et à diminuer la teneur en chlorure de vinyle résiduaire dans les produits de réaction.

En conséquence, l'invention concerne un procédé de fabrication de 1,1-difluoroéthane par réaction entre du fluorure d'hydrogène et du chlorure de vinyle en phase liquide, qui se caractérise en ce que la réaction est effectuée dans un milieu réactionnel comprenant au moins un composé choisi parmi le perchloroéthylène et le 1,1,1,3,3-pentafluorobutane. Le perchloroéthylène est préféré.

Par milieu réactionnel, on entend désigner l'ensemble du milieu liquide formé du composé susmentionné, du fluorure d'hydrogène et du chlorure de vinyle mis en oeuvre, des produits formés par la réaction du fluorure d'hydrogène et du chlorure de vinyle et, le cas échéant, d'autres additifs habituellement utilisés dans les procédés de fluoration des hydrocarbures halogénés, tels que des catalyseurs.

Dans le procédé selon l'invention, le milieu réactionnel contient habituellement au moins 20 % en poids du composé précité. De préférence, le milieu réactionnel en contient au moins 30 % en poids. De manière particulièrement préférée, il en contient au moins 40 % en poids. Les meilleurs résultats sont obtenus avec une teneur en composé précité dans le milieu réactionnel supérieure ou égale à 45 % en poids. En général, le milieu réactionnel contient au maximum 99,5 % en poids du composé précité. De préférence, il en contient au plus 98,5 %. De manière particulièrement préférée, il en contient au plus 95 %. De très bons résultats ont été obtenus avec une teneur en composé précité dans le milieu réactionnel inférieure ou égale à 90 %.

Le milieu réactionnel contient habituellement au moins 0,2 % en poids de fluorure d'hydrogène. De préférence, le milieu réactionnel contient au moins 1 % en poids de fluorure d'hydrogène. De manière particulièrement préférée, il en contient au moins 5% en poids. Les meilleurs résultats sont obtenus avec une teneur en fluorure d'hydrogène dans le milieu réactionnel supérieure ou égale à 10 % en poids. En général, la teneur en fluorure d'hydrogène dans le milieu réactionnel est au plus de 75 % en poids. De préférence, elle est inférieure ou égale à 60 %. De très bons résultats ont été obtenus avec une teneur en fluorure d'hydrogène dans le milieu réactionnel ne dépassant pas 50 % en poids. Une teneur en fluorure d'hydrogène dans le milieu réactionnel de 15 à 40 % est tout particulièrement avantageuse.

Plus la teneur en chlorure de vinyle dans le milieu réactionnel est faible, moins on forme de sous-produits lourds. La réactivité du chlorure de vinyle et du fluorure d'hydrogène dans le milieu réactionnel est telle que l'on peut travailler avec une teneur en chlorure de vinyle dans le milieu réactionnel de l'ordre de 0,003 % en poids. En pratique, on travaille de préférence avec une teneur en chlorure de vinyle dans le milieu réactionnel d'au moins 0,005 % en poids. Une teneur en chlorure de vinyle d'au moins 0,01 % en poids est particulièrement préférée. De très bons résultats ont été obtenus avec une teneur en chlorure de vinyle d'environ 0,03 % en poids. On travaille généralement avec une teneur en chlorure de vinyle inférieure à 15 % du poids du milieu réactionnel. De manière avantageuse, on travaille avec une teneur en chlorure de vinyle inférieure à 10 % en poids. De manière particulièrement avantageuse, elle ne dépasse à aucun moment 5 % en poids. De manière tout particulièrement avantageuse, on travaille avec une teneur en chlorure de vinyle inférieure à 2 % en poids.

Le milieu réactionnel peut contenir également divers additifs, en particulier un catalyseur d'hydrofluoration. La présence d'un catalyseur est souhaitable peur obtenir du 1,1-difluoroéthane dans des conditions industriellement exploitables. Comme catalyseurs utilisables, on peut citer les dérivés des métaux choisis parmi les métaux des groupes IIIa, IVa, IVb, Va, Vb et VIb du tableau périodique des éléments et leurs mélanges. On retient plus spécialement les dérivés de titane, de vanadium, de tantale, de molybdène, de tungstène, d'étain et d'antimoine. Les dérivés de l'étain, du titane, du molybdène et du tungstène sont préférés. Les dérivés de l'étain conviennent particulièrement bien. Comme dérivés des métaux, on utilise de préférence les halogénures, tels que les chlorures, les fluorures et les chlorofluorures, ainsi que les oxydes et les oxyhalogénures. Des catalyseurs particulièrement préférés pour préparer le 1,1-difluoroéthane par le procédé selon la présente invention sont les chlorures, les fluorures et les chlorofluorures d'étain. La mise en oeuvre de SnCl₄ dans le procédé selon l'invention est particulièrement avantageuse. En fonction des conditions de réaction, celui-ci peut être au moins partiellement fluoré in situ.

La quantité de catalyseur mise en oeuvre peut varier dans de larges limites. Habituellement, le catalyseur est utilisé à raison de 0,001 à 2 mole de catalyseur par kg de milieu réactionnel de préférence, de 0,01 à 1 mole par kg.

Lorsque le composé sus-mentionné est du 1,1,1,3,3-pentafluorobutane, un milieu réactionnel monophasique est généralement obtenu. Lorsque le composé sus-mentionné est du perchloroéthylène, un milieu réactionnel biphasique est le plus souvent obtenu, constitué d'une phase lourde, riche en composés organiques et d'une phase légère, riche en fluorure d'hydrogène.

Le procédé selon l'invention peut être réalisé selon un mode discontinu ou continu. Un mode de réalisation avantageux du procédé consiste à introduire le chlorure de vinyle et le fluorure d'hydrogène réactifs en continu dans le réacteur contenant ledit composé et, éventuellement un excès de fluorure d'hydrogène et un catalyseur et à soutirer le 1,1-difluoroéthane en continu hors du milieu réactionnel.

Dans le procédé selon l'invention, on introduit avantageusement le fluorure d'hydrogène dans un rapport molaire fluorure d'hydrogène / chlorure de vinyle au moins égal à 2. Le plus souvent, ce rapport molaire ne dépasse pas 20. De préférence, Il ne dépasse pas 5. D'excellents résultats ont été obtenus avec un rapport molaire de 2 ou légèrement supérieur, par exemple de 2,05 à 2,5.

Dans le procédé selon l'invention, le fluorure d'hydrogène et le chlorure de vinyle peuvent être introduits dans le milieu réactionnel indifféremment à l'état liquide ou à l'état vapeur. En raison de la présence du composé précité, le chlorure de vinyle peut même être introduit dans le réacteur à l'état liquide sans entraîner la formation de quantités importantes de sous-produits lourds.

La durée de la réaction nécessaire pour assurer un rendement optimal en 1,1-difluoroéthane est variable en fonction des conditions opératoires et, en pratique, peut être évaluée facilement par voie expérimentale. Lorsque le procédé est effectué en continu, le temps de séjour conventionnel, c'est-à-dire le rapport entre le volume de milieu réactionnel contenu dans le réacteur et le débit total de chlorure de vinyle et de fluorure d'hydrogène à l'état liquide est généralement de 0,1 à 5 heures.

Le procédé selon l'invention peut être réalisé dans de larges gammes de températures et de pressions, dans lesquelles le milieu réactionnel est liquide. Généralement, la température à laquelle la réaction est réalisée est au moins de 40 °C et ne dépasse pas 130 °C. De préférence, elle est d'au moins 50 °C et ne dépasse pas 120 °C. Généralement, on travaille à une pression d'au moins environ 2 bar. Une pression d'au moins environ 5 bar est préférée. Une pression d'au moins environ 8 bar est particulièrement préférée. Le plus souvent, cette pression ne dépasse pas environ 50 bar, les pressions inférieures ou égales à environ 30 bar étant spécialement recommandées.

Selon un mode de réalisation particulièrement préféré de l'invention, la température et la pression de réaction sont réglées de manière, d'une part à assurer le maintien en phase liquide du milieu réactionnel d'autre part, à permettre au 1,1-difluoroéthane et au chlorure d'hydrogène co-produit de quitter le milieu réactionnel sous forme gazeuse. Pour ce faire, on travaille généralement à des températures comprises entre 60 et 120 °C et à des pressions comprises entre 2 et 30 bar. Des températures comprises entre 70 et 100 °C et des pressions comprises entre 7 et 15 bar se sont révélées avantageuses.

Dans ce mode de réalisation particulièrement préféré de l'invention, le milieu gazeux que l'on recueille contient, outre le 1,1-difluoroéthane et le chlorure d'hydrogène, un peu de fluorure d'hydrogène, éventuellement un peu de 1-chloro-1-fluoroéthane produit par fluoration incomplète du chlorure de vinyle et, éventuellement, un peu du composé précité . Typiquement, il ne contient pas plus de 100 ppm de chlorure de vinyle. Lorsque le milieu réactionnel contient du perchloroéthylène, une teneur en chlorure de vinyle ne dépassant pas 30 ppm peut même être obtenue. Le milieu gazeux peut être soumis à une ou plusieurs étapes de séparation par des techniques connues en soi, de manière à recueillir le 1,1-difluoroéthane sous forme substantiellement pure. Le chlorure d'hydrogène, le fluorure d'hydrogène et le composé précité peuvent être séparés par exemple par distillation. Aux faibles concentrations obtenues par le procédé selon l'invention, le chlorure de vinyle résiduaire peut facilement être éliminé par adsorption sur charbon actif.

Le procédé selon l'invention peut être mis en oeuvre dans tout type de réacteur ou d'appareillage permettant de réunir les conditions décrites et en particulier de résister à la pression et au fluorure d'hydrogène. Le plus souvent, le procédé selon l'invention est réalisé dans un réacteur équipé d'un dispositif de soutirage d'un courant gazeux, par exemple dans un réacteur surmonté d'une colonne et d'un condenseur de reflux. Ce dispositif permet, par un réglage adéquat, de soutirer en phase gazeuse le 1,1-difluoroéthane et le chlorure d'hydrogène produits, tout en maintenant dans le réacteur les autres constituants du milieu réactionnel.

Le procédé selon l'invention présente certains avantages appréciables par rapport au procédé décrit dans la demande de brevet EP-A-0637579. Il permet notamment d'obtenir du 1,1-difluoroéthane avec une sélectivité et un rendement bien supérieurs à ceux obtenus dans les procédés connus. En particulier, il génère beaucoup moins de sous-produits lourds et améliore très nettement la stabilité du catalyseur, ce qui dispense de l'obligation de procéder fréquemment à une purge d'une fraction du milieu réactionnel et à l'introduction de catalyseur frais. De plus, l'accroissement important de réactivité du chlorure de vinyle envers le fluorure d'hydrogène observé dans le procédé selon l'invention confère à celui-ci une productivité largement supérieure à celle des procédés connus et permet en outre d'obtenir directement un mélange gazeux à la sortie du réacteur renfermant typiquement seulement de 10 à 100 ppm de chlorure de vinyle, ce qui évite des traitements difficiles et coûteux de purification du 1,1-difluoroéthane.

En variante, dans le procédé selon l'invention, le chlorure de vinyle peut être remplacé par du 1,1-dichloroéthane ou du 1-chloro-1-fluoroéthane.

Les exemples ci-après illustrent l'invention de manière non limitative.

### Exemple 1 (comparaison)

Dans un autoclave de 0,5 l en alliage HASTELLOY® B2, équipé d'un agitateur à pales et surmonté d'un condenseur à double enveloppe, préalablement mis sous vide, on a introduit à température ambiante, 108 g de 1,1-difluoroéthane, conformément au procédé décrit dans la demande EP-A-0637579, 15 g de SnCl₄ et 135 g de fluorure d'hydrogène. L'autoclave a ensuite été plongé dans un bain d'huile thermostatisé, et chauffé jusqu'à l'obtention d'une température de 85 à 90 °C. La pression dans l'autoclave et le condenseur a été régulée à 12 bar. L'autoclave a ensuite été alimenté en continu en fluorure d'hydrogène et en chlorure de vinyle, à un débit en chlorure de vinyle de 15 g/h et un rapport molaire fluorure d'hydrogène / chlorure de vinyle de 2. Le condenseur a été maintenu à une température de 30 °C, le condensat étant renvoyé en continu à l'autoclave et les effluents gazeux neutralisés dans un scrubber à l'aide d'une solution concentrée d'hydroxyde de potassium. Le suivi de la réaction a été effectué sur base de l'analyse de prélèvements réguliers du milieu liquide dans l'autoclave, de l'analyse de la composition des effluents gazeux en sortie du scrubber, ainsi que de l'analyse des chlorures et des fluorures récoltés au scrubber.

Les résultats les plus significatifs sont présentés dans le tableau unique. Il s'agit de la durée de réaction avant observation d'une diminution de l'activité catalytique, de la proportion pondérale de sous-produits lourds formés par rapport au chlorure de vinyle (VC) transformé (sélectivité en sous-produits lourds) et de la teneur minimale en chlorure de vinyle observée dans les effluents gazeux ([VC]ₘᵢₙ). La diminution de l'activité catalytique se marque par une diminution de la productivité en 1,1-difluoroéthane, coïncidant avec une augmentation de la concentration en chlorure de vinyle dans les effluents gazeux et une accumulation de 1-chloro-1-fluoroéthane et de 1,1-dichloroéthane dans le milieu réactionnel.

### Exemple 2 (comparaison)

L'essai de l'exemple 1 a été répété en remplaçant le 1,1-difluoroéthane par du 1,2,3-trichloropropane. La pression a été régulée à 10 bar. Les résultats sont repris dans le tableau unique.

### Exemples 3 et 4 (selon l'invention)

L'essai de l'exemple 1 a été répété en remplaçant le 1,1-difluoroéthane par du 1,1,1,3,3-pentafluorobutane (exemple 3; pression régulée à 12 bar) ou du perchloroéthylène (exemple 4; pression régulée à 10 bar). Les résultats sont également repris dans le tableau unique.

L'analyse du tableau met en évidence le progrès réalisé en incorporant du 1,1,1,3,3-pentafluorobutane ou du perchloroéthylène au milieu réactionnel, conformément à l'invention. Ce progrès se manifeste par une nette amélioration de la durée de vie du catalyseur et par une diminution significative de la quantité de sous-produits lourds formés et de la teneur en chlorure de vinyle résiduel dans les produits de la réaction.

**TABLEAU**

| N° exemple | Nature du composé | Durée réaction stable (h) | sous-produits lourds (g/kg VC converti) | [VC]ₘᵢₙ (ppm) |
|---|---|---|---|---|
| 1 (C) | 1,1-difluoroéthane | 50 | 50 | > 1000 |
| 2 (C) | 1,2,3-trichloropropane | 35 | 25 | 1400 |
| 3 | 1,1,1,3,3-pentafluorobutane | 100 | 6 | 100 |
| 4 | perchloroéthylène | > 200 | 1 | 30 |

## Revendications

1. Procédé de fabrication de 1,1-difluoroéthane par réaction entre du fluorure d'hydrogène et du chlorure de vinyle en phase liquide, caractérisé en ce que la réaction est effectuée dans un milieu réactionnel comprenant au moins un composé choisi parmi le perchloroéthylène et le 1,1,1,3,3-pentafluorobutane.

2. Procédé selon la revendication 1, dans lequel ledit composé est le perchloroéthylène.

3. Procédé selon la revendication 1 ou 2, dans lequel le milieu réactionnel contient de 20 à 99,5 % en poids dudit composé.

4. Procédé selon une quelconque des revendications 1 à 3, dans lequel le milieu réactionnel contient de 0,2 à 75 % en poids de fluorure d'hydrogène.

5. Procédé selon une quelconque des revendications 1 à 4, dans lequel le milieu réactionnel contient de 0,003 à 15 % en poids de chlorure de vinyle.

6. Procédé selon l'une quelconque des revendications 1 à 4, dans lequel le milieu réactionnel contient de 0,001 à 2 mole par kg d'un catalyseur d'hydrofluoration choisi parmi les dérivés des métaux des groupes IIIa, IVa, IVb, Va, Vb et VIb du tableau périodique des éléments.

7. Procédé selon l'une quelconque des revendications 1 à 6, dans lequel le rapport molaire entre le fluorure d'hydrogène et le chlorure de vinyle mis en oeuvre est d'au moins 2 et ne dépasse pas 20.

8. Procédé selon l'une quelconque des revendications 1 à 7, dans lequel la réaction est réalisée à une température d'au moins 40 °C et ne dépassant pas 130 °C et sous une pression d'au moins 2 bar et ne dépassant pas 50 bar.

9. Procédé selon l'une quelconque des revendications 1 à 8, dans lequel on soutire en continu le 1,1-difluoroéthane hors du milieu réactionnel.

10. Procédé selon la revendication 9, dans lequel on soutire le 1,1-difluoroéthane et le chlorure d'hydrogène produits en phase gazeuse.

11. Procédé selon l'une quelconque des revendications 1 à 10, dans lequel au moins une partie du chlorure de vinyle est remplacée par du 1,1-dichloroéthane ou du 1-chloro-1-fluoroéthane.

## Claims

1. Process for the manufacture of 1,1-difluoroethane by reaction between hydrogen fluoride and vinyl chloride in the liquid phase, characterized in that the reaction is carried out in a reaction medium comprising at least one compound chosen from perchloroethylene and 1,1,1,3,3-pentafluorobutane.

2. Process according to Claim 1, in which the said compound is perchloroethylene.

3. Process according to Claim 1 or 2, in which the reaction medium contains from 20 to 99.5 % by weight of the said compound:

4. Process according to any one of Claims 1 to 3, in which the reaction medium contains from 0.2 to 75 % by weight of hydrogen fluoride.

5. Process according to any one of Claims 1 to 4, in which the reaction medium contains from 0.003 to 15 % by weight of vinyl chloride.

6. Process according to any one of Claims 1 to 4, in which the reaction medium contains from 0.001 to 2 mol per kg of a hydrofluorination catalyst chosen from derivatives of the metals from groups IIIa, IVa, IVb, Va, Vb and VIb of the Periodic Table of the Elements.

7. Process according to any one of Claims 1 to 6, in which the molar ratio between the hydrogen fluoride and the vinyl chloride used is at least 2 and does not exceed 20.

8. Process according to any one of Claims 1 to 7, in which the reaction is performed at a temperature of at least 40°C and not exceeding 130°C and at a pressure of at least 2 bar and not exceeding 50 bar.

9. Process according to any one of Claims 1 to 8, in which the 1,1,-difluoroethane is continuously withdrawn from the reaction medium.

10. Process according to Claim 9, in which the 1,1-difluoroethane and the hydrogen chloride produced are withdrawn in the gas phase.

11. Process according to any one of Claims 1 to 10, in which at least a part of the vinyl chloride is replaced by 1,1-dichloroethane or 1-chloro-1-fluoroethane.

## Patentansprüche

1. Verfahren zur Herstellung von 1,1-Difluorethan durch Reaktion zwischen Fluorwasserstoff und Vinylchlorid in flüssiger Phase, dadurch gekennzeichnet, daß die Reaktion in einem Reaktionsmedium ausgeführt wird, das wenigstens eine Verbindung umfaßt, die unter Perchlorethylen und 1,1,1,3,3-Pentafluorbutan ausgewählt ist.

2. Verfahren gemäß Anspruch 1, bei dem besagte Verbindung Perchlorethylen ist.

3. Verfahren gemäß Anspruch 1 oder 2, bei dem das Reaktionsmedium 20 bis 99,5 Gew.-% besagter Verbindung enthält.

4. Verfahren gemäß einem der Ansprüche 1 bis 3, bei dem das Reaktionsmedium 0,2 bis 75 Gew.-% Fluorwasserstoff enthält.

5. Verfahren gemäß einem der Ansprüche 1 bis 4, bei dem das Reaktionsmedium 0,003 bis 15 Gew.-% Vinylchlorid enthält.

6. Verfahren gemäß einem der Ansprüche 1 bis 4, bei dem das Reaktionsmedium 0,001 bis 2 Mol pro kg eines Hydrofluorierungskatalysators, der unter den Derivaten der Metalle der Gruppen IIIa, IVa, IVb, Va, Vb und VIb des Perioden-Systems der Elemente ausgewählt ist, enthält.

7. Verfahren gemäß einem der Ansprüche 1 bis 6, bei dem das eingesetzte Molverhältnis zwischen Fluorwasserstoff und Vinylchlorid wenigstens 2 beträgt und 20 nicht übersteigt.

8. Verfahren gemäß einem der Ansprüche 1 bis 7, bei dem die Reaktion bei einer Temperatur, die wenigstens 40 °C beträgt und 130 °C nicht übersteigt, und unter einem Druck, der wenigstens 2 bar beträgt und 50 bar nicht übersteigt, durchgeführt wird.

9. Verfahren gemäß einem der Ansprüche 1 bis 8, bei dem man das 1,1-Difluorethan kontinuierlich aus dem Reaktionsmedium entnimmt.

10. Verfahren gemäß Anspruch 9, bei dem man 1,1-Difluorethan und Chlorwasserstoff, die in der Gasphase hergestellt wurden, entnimmt.

11. Verfahren gemäß einem der Ansprüche 1 bis 10, bei dem wenigstens ein Teil des Vinylchlorids durch 1,1-Dichlorethan oder 1-Chlor-1-fluorethan ersetzt wird.
